# EUROPEAN PATENT SPECIFICATION

(11) **EP 1 051 432 B1**
(45) Date of publication and mention of the grant of the patent: **24.01.2007**
(21) Application number: 99959535.8
(22) Date of filing: 08.12.1999
(51) Int. Cl.: C07K 14/435, C07K 14/315, C07K 16/46, G01N 33/563

(54) **METHOD FOR REDUCING IMMUNOGENICITY OF PROTEINS**
VERFAHREN ZUR VERMINDERUNG DER IMMUNOGENITÄT VON PROTEINEN
MODIFICATION DE L'IMMUNOGENICITE DE PROTEINES

(30) Priority: 08.12.1998 GB 9826925; 02.02.1999 GB 9902139
(43) Date of publication of application: 15.11.2000
(73) Proprietor: Biovation Limited, Aberdeen AB10 1DQ (GB)
(72) Inventor: CARR, Francis Joseph, Biovation Ltd., Aberdeen AB22 8GU (GB); ADAIR, Fiona Suzanne, Biovation Limited, Aberdeen AB22 8GU (GB); HAMILTON, Anita Anne, Biovation Limited, Aberdeen AB22 8GU (GB); CARTER, Graham, Biovation Limited, Aberdeen AB22 8GU (GB)
(74) Representative: Benz, Jürgen
(86) International application number: PCT/GB1999/004119
(87) International publication number: WO 2000/034317

(56) References cited:
- EP-A- 0 699 755
- EP-A- 0 721 982
- WO-A-92/10755
- WO-A-98/52976
- J. EXP. MED, vol. 182, November 1995 (1995-11), pages 1481-1491,
- SCAND. J. IMMUNOL, vol. 36, 1992, pages 653-660,
- NOLTE K U; GÜNTHER G; VON WUSSOW P: "Epitopes recognized by neutralizing therapy-induced human anti-interferon-alpha antibodies are localized within the N-terminal functional domain of recombinant interferon-alpha 2" EUROPEAN JOURNAL OF IMMUNOLOGY, vol. 26, no. 9, 1996, pages 2155-2159,

## Description

THE PRESENT INVENTION relates to proteins to be administered especially to humans particularly for therapeutic use but also for use in diagnostic tests. The invention particularly provides for proteins which are modified to be less immunogenic than the unmodified counterpart when used *in vivo.*

The invention particularly addresses the clinical need for a process by which the natural tendency of the host to mount an immune response against an administered protein is substantially reduced or eliminated. There are several examples where the administration of protein molecules offers therapeutic benefit. However the benefit is greatly reduced particularly where multiple doses of the therapeutic protein are required as the recipient immune system recognises and accelerates the elimination of the incoming therapeutic protein.

There are a number of therapeutic proteins whose therapeutic use is curtailed on account of their immunogenicity in man. For example, when murine antibodies are administered to patients who are not immunosuppressed, a majority of patients mount an immune reaction to the foreign material by making human anti-murine antibodies (HAMA). There are two serious consequences. First, the patient's anti-murine antibody may bind and clear the therapeutic antibody or immunoconjugate before it has a chance to bind to the tumour and perform its function. Second, the patient may develop an allergic sensitivity to the murine antibody and be at risk of anaphylactic shock upon any future exposure to murine immunoglobulin.

Several techniques have been employed to address the HAMA problem and thus enable the use in humans of therapeutic monoclonal antibodies (see, for example WO-A-8909622, EP-A-0239400, EP-A-0438310, WO-A-9109967). A common aspect of these methodologies has been the introduction into the therapeutic antibody, which in general is of rodent origin, of significant tracts of sequence identical to that present in human antibody proteins. Such alterations are also usually coupled to alteration of particular single amino acid residues at positions considered critical to maintaining the antibody-antigen binding interaction. For antibodies, this process is possible owing to the very high degree of structural (and functional) conservatism between antibody molecules of different species. However for potentially therapeutic proteins where no structural homologue may exist in the host species (e.g. human) for the therapeutic protein, such processes are not applicable. Furthermore, these methods have assumed that the general introduction of human sequence will render the re-modelled antibody non-immunogenic. It is known however, that certain short peptide sequences ("T cell epitopes') can be released during the degradation of proteins within cells and subsequently presented by molecules of the major histocompatability complex (MHC) in order to trigger the activation of T cells. For peptides presented by MHC class II, such activation of T cells can then give rise to an antibody response by direct stimulation of B cells to produce such antibodies. None of the previous methods have addressed the elimination or avoidance of such epitopes in the final therapeutic molecule as a means to reduce or eliminate the antibody response against proteins. Nor have previous methods considered the elimination of peptides presented by MHC class I which can trigger a cytotoxic T cell response leading to cell killing. Such killing of cells causes the release of cellular components including proteins which can, in turn, activate specialist cells which are highly active in protein processing and MHC presentation and also in release of inflammatory cytokines as a result of such activation. As a result, an inflammatory environment can be created which promotes the more active uptake and processing of the protein for therapeutic use thus facilitating induction of an antibody response against the protein.

WO 98/52975 teaches the removal of T-cell epitopes from proteins, preferably therapeutic antibodies, which are heterologous to a specific host organism, such as a human.

WO 92/10755 is concerned with a method for B-cell epitope mapping using immunological and proteochemical methods, and with corresponding epitopes that are changed by mutation.

EP 0721 982 provides variants of staphylokinase, a bacterium protein, which were modified with respect to existing B-cell epitopes.

EP 0699755 is directed to a method for modifying immunoglobulin V region domains of a heterologous antibody by using different humanization techniques.

Fedoseyeva et al. (J. Exp. Med. 182, 1995, 1481-1491) reported that certain self-peptides processed from autologous proteins by presentation to autoreactive T-cells were immunogenic in syngeneic animals. Tiarks et al. (Scand. J. Immunol., 36, 1992,, 653-660) teaches the necessity of modifying T-cell epitopes within Factor VIII without significantly altering its biological function.

Another scientific paper (Nolte et al., 1996, Eur. J. Immunol., 26,2155-2159) reported that patients receiving interferon alpha 2 (IFN2) may develop anti-IFN2 antibodies, which recognize B-cell epitopes that have to be identified and eliminated.

The elimination of T cell epitopes from proteins has been previously disclosed (see WO-A-9852976) whereby such potential T cell epitopes are defined as any peptide within the protein sequence with the ability to bind to MHC class II molecules. Such potential opitopcs are measured by any computational or physical method to establish MHC binding. Implicit in the term "T cell epitope" is an epitope which is recognised by the T cell receptor and which can at least in principle cause the activation of these T cells. It is however usually understood that certain peptides which are found to bind to MHC class II molecules may be retained in a protein sequence because such peptides are recognised as "self" within the organism into which the final protein is administered.

In practice, soluble proteins introduced into autologous organisms do sometimes trigger an immune response resulting in development of host antibodies which bind to the soluble protein. One example is interferon alpha 2 to which a percentage of human patients make antibodies despite the fact that this protein is produced endogenously.

The present invention is based on the discovery by the inventors that MHC-binding peptides within autologous proteins such as interferon alpha 2 can trigger immune responses in live organisms to those proteins oven when the specific protein is endogenously produced. A possible explanation for this is that the doses of such proteins administered are much higher than normal providing MHC-peptide formation which can activate T cells. Alternatively, the physiological environment into which such administered proteins are presented is one favourable to efficient antigen processing and peptide-MHC formation at higher levels than usually encountered, for example in inflammatory situations.

According to a specific aspect of the invention there is provided a method of rendering human interferon alpha 2 (IFN2) non-immunogenic or less immunogenic to a human, the method comprising:
(a) determining at least part of the amino acid sequence of mature human IFN2;
(b) identifying potential T-cell epitopes by analysing said sequence for the presence of human MHC class II binding motifs, and
(c) eliminating from said amino acid sequence at least one of said T-cell epitopes identified according to step b), thereby reducing the immunogenicity of IFN2 when exposed to the immune system of a human.

In step (c), one or more actual epitopes or potential epitopes may be eliminated.

Therefore, the present invention provides a modified method for creating human IFN2 which triggers a reduced or absent immune response whereby one or more MHC binding peptides which are also found in the autologous organism's endogenous protein are modified to reduce or eliminate binding to MHC molecules. Thus, in practice, no assumption is made about which peptides might be tolerated by the organism (unless data is available which indicates that tolerance is in place). Overall, the invention encompasses the creation of IFN2 protein molecules with reduced or absent immunogenicity in live organisms where one or more peptides which bind to MHC Class II molecules are eliminated from the protein molecule.

The aspect of the invention described above is preferred to the alternative strategy of separately immunising the organism with such peptides in order to induce tolerance or the alternative of administering analogues or fragments of the protein which resist binding or uptake into cells (especially antigen-presenting cells), for example by elimination of the cell binding site in the protein. Tolerising the organism to the peptide in this way (*e.g*. the use of MHC binding peptides identified from a syngeneic protein which can be used to induce tolerance in the host prior to administration of the entire protein) would require two therapeutic molecules, one to perform the main protein function and the other to induce the tolerance, which would not be favoured from the regulatory point of view.

Any method of identifying - in which term is included predicting - one or more potential T cell epitopes can be used in the invention. Acceptable methods may be computational or physical and include measuring of the binding of MHC-peptide complexes to T cell receptors, the testing of potential T cell epitopes in transgenic mice expressing human MHC molecules, the testing of potential T cell epitopes in mice reconstituted with human antigen-presenting cells and T cells in place of their endogenous cells, and the testing of potential T cell epitopes for stimulation of T cells in *vitro* via presentation of peptides on MHC molecules using syngeneic antigen-presenting cells.

As well as identifying in the amino acid sequence one or more potential T cell epitopes which are found in human IFN2, a method of the invention may, and typically will, involve additionally identifying and eliminating one or more potential T cell epitopes which are not found in an endogenous human IFN2 of a human, since such epitopes are even more likely to be immunogenic.

The present invention is based on a new concept in development of therapeutic proteins. It has been recognised previously that, with exogenous T cell dependent proteins, helper T cell epitopes are important to development of a significant immune response to the autologous protein. Such T cell epitopes function through the internal processing of the protein releasing peptides which complex with MHC class II molecules and which are then presented on the surface of appropriate cells for potential binding to receptors on T cells. However, it is very difficult to predict which peptides within the protein will be processed appropriately such that MHC binding can occur and it is also a factor that many processed peptides will not bind to all allotypic variants of MHC class II molecules (MHC restriction). Furthermore, in any given living organism, it is difficult to predict whether a T cell response will actually be triggered by a given peptide presented on MHC class II because T cells may have been tolerised to such epitopes or may not have the repertoire of T cell receptors to bind to the MHC class II-peptide complex. Due to these complicating factors, it has previously been impractical to develop proteins with reduced or absent immunogenicity because of the difficulty in predicting actual T cell epitopes. The invention herein primarily takes, the new approach of creating improved therapeutic proteins such as human IFN2 by removal of potential rather than actual T cell epitopes (usually defined by binding to MHC molecules) such that certain peptides within the molecule which are not actually immunogenic may be altered in addition to those immunogenic peptides. For a therapeutic IFN2 preferably all of the potential T cell epitopes are removed whilst retaining activity of the protein. Preferably, this involves judicious choice of the amino acid substitutions enabling removal of the T cell epitopes and usually will involve the testing of a range of variant molecules with different amino acid substitutions.

Not all identified potential T cell epitopes which are found in human IFN2 need be eliminated. For example, immune responses in general are not mounted to autologous circulating proteins, such as immunoglobulins and other serum proteins such as serum albumin. So potential T cell epitopes which are found for example in germline immunoglobulin variable region protein sequences of the given species (which will generally be human) may sometimes be ignored. However, an epitope considered as not generally available to the immune system to gain tolerance may be identified as a potential epitope for elimination according to the method of the present invention.

The invention therefore provides human IFN2 proteins which have been altered to reduce their immunogenicity as well as a general method of altering said protein to reduce its immunogenicity. A major principle of the present invention is that proteins are altered by identification of potential T cell epitopes and their subsequent alteration within the proteins in order to eliminate such potential epitopes.

After elimination of one or more T cell epitopes, the protein may be tested for desired activity. It may retain its full activity, or it may retain a sufficient proportion of its original activity to be useful. In some circumstances, the activity may be altered either beneficially or at least in an acceptable way. Proteins entirely lacking in useful function post modification may be discarded.

A typical protocol within the general method of the present invention comprises the following steps:
I. Determining the amino acid sequence of human IFN2 or a part thereof (if modification only of a part is required);
II. Identifying potential T cell epitopes within the amino acid sequence of the protein by any method including determination of the binding of peptides to MHC molecules, determination of the binding of peptide:MHC complexes to the T cell receptors from the species to receive the therapeutic protein, testing of the protein or peptide parts thereof using transgenic animals with the MHC molecules of the species to receive the therapeutic protein, or testing with transgenic animals reconstituted with immune system cells from the species to receive the therapeutic protein;
III. By genetic engineering or other methods for producing modified proteins, altering the IFN2 protein to remove one or more of the potential T cell epitopes and producing such an altered protein for testing;
IV. (optionally) Within step III., altering the IFN2 protein to remove one or more of the potential B cell epitopes;
V. Testing altered proteins with one or more potential T cell epitope (and optionally B cell epitopes) removed in order to identify a modified IFN2 protein which has retained all or part of its desired activity but which has lost one or more T cell epitopes.

Potential T-cell epitopes herein are defined as specific peptide sequences which either bind with reasonable efficiency to MHC class II molecules (or their equivalent in a non-human species), or which in the form of peptide:MHC complexes bind strongly to the T cell receptors from the species to receive the therapeutic protein or which, from previous or other studies, show the ability to stimulate T-cells via presentation on MHC class II. The method of the present invention recognises that an effective T cell-dependant immune response to a foreign protein requires activation of the cellular arm of the immune system. Such a response requires the uptake of the therapeutic (foreign) protein by antigen presenting cells (APCs). Once inside such cells, the protein is processed and fragments of the protein form a complex with MHC class II molecules and are presented at the cell surface. Should such a complex be recognised by binding of the T cell receptor from T-cells, such cells can be, under certain conditions, activated to produce stimulatory cytokines. The cytokines will elicit differentiation of B-cells to full antibody producing cells. In addition, such T cell responses may also mediate other deleterious effects on the patient such as inflammation and possible allergic reaction.

It is understood that not all peptide sequences will be delivered into the correct MHC class II cellular compartment for MHC class II binding or will be suitably released form a larger cellular protein for subsequent MHC class II binding. It is further understood that even such peptides which are presented by MHC class II on the surface of APCs may not elicit a T-cell response for reasons including a lack of the appropriate T-cell specificity, tolerance by the immune system to the particular peptide sequence or the low affinity of the MHC-peptide complex for T cell receptor.

The present invention provides for removal human potential T-cell epitopes from therapeutic human IFN2 protein whereby the primary sequence of the therapeutic protein can be analysed for the presence of MHC class II binding motifs by any suitable means. For example, a comparison may be made with databases of MHC-binding motifs such as, by searching the "motifs" database at the world-wide web site http://wehih.wehi.edu.au/mhcpep/ (formerly wehil.wehi.edu.au or wchi.wehi.edu.au). Alternatively, MHC class II binding peptides may bo identified using computational threading methods such as those devised by Altuvia *et al. ((J. Mol. Biol.* **249** 244-250 (1995))). Similar methods may be used to identify and remove epitopes which bind to MHC class I.

Having identified potential given species (*e*.*g*. human) T-cell epitopes, these epitopes are then eliminated by alteration of one or more amino acids, as required to eliminate the T-cell epitope. Usually this will involve alteration of one or more amino acids within the T-coll epitope itself. This could involve altering an amino acid adjacent to the epitope in terms of the primary structure of the protein or one which is not adjacent in the primary structure but is adjacent in the secondary structure of the molecule. The usual alteration contemplated will be amino acid substitution, but in certain circumstances amino acid deletion or addition will be appropriate. In some instances amino acid substitutions may be made to prevent proteolytic cleavage of a protein and thereby inhibit binding of a peptide to MHC class II. Examples of protease cleavage sites include those reported by van Noort & van der Drift *(J. Biol. Chem.* 264 14159-14164 (1989)) and van Noort *et al.* (*Eur. J. Immunol.* 21 1989-1996 (1991)). Examples of amino acids which prevent protease digestion have been identified e.g. Kropshofer *et al*., *J*. *Immunol.* 151 4732-4742 (1993).

In the method of the present invention, usually a number of variants of altered IFN2 proteins will be produced and tested for retention of the desired activity. Where it is desirable to maximise the removal of potential T cell epitopes from the protein, it will be common to create a range of variants including some with potential T cell epitopes remaining in the molecule. It will be recognised that with certain protein molecules, it is difficult to radically alter the molecule and to retain full activity and so judicious use of molecular modelling of variants and the testing of a maximum number of variants is desirable. For molecular modelling, standard commercially available software packages can be used to model the protein structure using as starting point either of a crystal structure or a model built from homology or protein folding prediction. Information on parts of the protein involved in imparting the molecule with its activity will assist in modelling variants enabling best choice of altered amino acids which remove a potential T cell epitope (or optional B cell epitope) whilst retaining activity.

In the method of the present invention where a number of variants of altered human IFN2 proteins will be produced and tested for retention of the desired activity, it is desirable to have a high-throughput method for screening large numbers of variants. Such methods include in vivo methods for expression of gene variants in cells such as bacteria with rapid isolation of the proteins for example using an antibody to a conserved region of the proteins, or via a protein purification tag included in the protein sequence. As an alternative, in *vitro* methods such as *in vitro* transcription and translation can be used in some cases. Where the protein binds to another molecule, display methods such as phage display and ribosome display can be used in order to select out variants which retain binding activity.

In the practice of the present invention, specific amino acid alterations can be conducted by recombinant DNA technology, so that the final molecule may be prepared by expression from a recombinant host using established methods. However, the use of protein chemistry or any other means of molecular alteration is not excluded in the practice of the invention. Whilst the present invention provides a method for removal of potential T cell epitopes (and optional B cell epitopes) from protein molecules, the method does not exclude testing of variant molecules produced within the invention for actual T cell epitope activity including testing of the protein or peptide parts thereof using transgenic animals with the MHC molecules of the species to receive the therapeutic protein, or testing with transgenic animals reconstituted with immune system cells from the species to receive the therapeutic protein.

Whilst such methods are not yet routine, *in vitro* T cell assays can be undertaken whereby the IFN2 protein can be processed and presented on MHC class II by appropriate antigen-presenting cells (APCs) to syngeneic T cells. T cell responses can be measured by simple proliferation measurements (especially if the APCs have been irradiated or otherwise treated to prevent proliferation) or by measuring specific cytokine release. In order to account for different MHC class II allotypes, a range of in vivo assays will usually be required in order to broadly test for T cell epitopes. Alternatively, transgenic animals equipped with human (or the desired species) MHC class II molecules could be used to test for T cell epitopes especially where the host MHC class II repertoire has been removed and especially where one or more other host accessory molecules in APC/T cell interaction have also been replaced with human (or the desired species) such as CD4 on T cells.

In a further aspect, the invention provides human IFN2 molecules resulting from method as described above. Such molecules may be useful for treating or preventing a disease or condition. The invention also extends to the use of such IFN2 molecules in in vivo and in *vitro* diagnosis and for human or veterinary use. Preferred features of each aspect of the invention are as for each other aspect, *mutatis mutandis.*

The invention is illustrated but not limited by the following examples. The examples refer to the following figures:
FIGURE 1 shows the protein sequence of mature human interferon 2 ; and
FIGURE 2 shows the protein sequence of an altered human interferon 2 molecule.

### Example

In the present example the human interferon α2 protein is analysed for the presence of potential MHC binding motifs and a method disclosed for the removal of a number of these from the molecule.

Interferon alpha 2 (INF2) is an important glycoprotein cytokine expressed by activated macrophages. The protein has antiviral activity and stimulates the production of at least two enzymes; a protein kinase and an oligoadenylate synthetase, on binding to the interferon alpha receptor in expressing cells. The mature INF2 protein is single polypeptide of 165 amino acids produced by post-translational processing of a 188 amino acid pre-cursor protein by cleavage of a 23 amino acid signal sequence from the amino terminus.

The protein has considerable clinical importance as a broad spectrum anti-viral, antiproliferative and immunomodulating agent. Recombinant and other preparations of INA2 have been used therapeutically in a variety of cancer and viral indications in man (reviewed in Sen, G.G. and Lengyel P, *J*. *Biol. Chem.* **267** 5017-5020 (1992)). However despite very significant therapeutic benefit to large numbers of patients, resistance to therapy in certain patients has been documented and one important mechanism of resistance has been shown to be the development of neutralising antibodies detectable in the serum of treated patients (Quesada, J.R. *et al*., *J. Clin. Oncology* 31522-1528 (1985); Stein R.G. *et al*., *New Eng. J. Med.* **318** 1409-1413 (1988); Russo, D. *et al*., *Br. J. Haem.* **94** 300-305 (1996); Brooks M.G. *et al*., *Gut* **30** 1116-1122 (1989)). An immune response in these patients is mounted to the therapeutic interferon despite the fact that a molecule of at least identical primary structure is produced endogenously in man. In the present example, an interferon alpha 2 molecule with reduced potential immunogenicity in man is presented.

### Method for identification of potential MHC class II binding motifs in human interferon alpha 2:

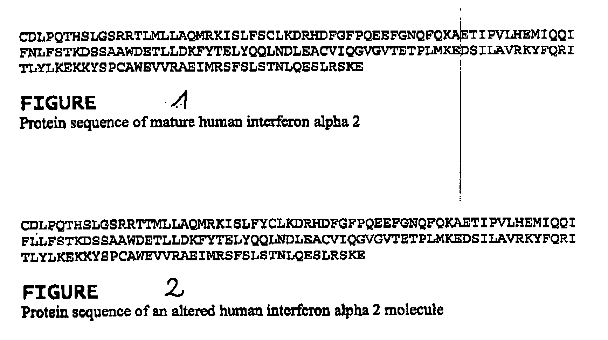

The sequence of INF2 was identified from the GenBank database. The sequence with accession number P01563 was used throughout. The protein sequences of the mature INF2 protein was identified and the sequence excluding the first 23 amino acid signal peptide analysed for the presence of potential MHC class II binding motifs (Figure 1). Analysis was conducted by computer using MPT ver1.0 software (Biovation, Aberdeen, UK). This software package conducts "peptide threading" according to the methods disclosed in WO-A-9859244. The software is able to provide an index of potential peptide binding to 18 different MHC class II DR alleles covering greater than 96% of the HLA-DR allotypes extant in the human population.

Results of the "peptide threading" process on the INF2, indicate the presence of a total of 18 individual potential epitopes. The epitopes map to five distinct clusters of overlapping epitopes encompassing residues 7-40 (cluster 1), residues 45-70 (cluster 2), residues 79-103 (cluster 3), residues 108-132 (cluster 4) and residues 140-163 (cluster 5). Each of the five clusters contain 5, 3, 4, 3 and 3 potential epitopes respectively.

In order to prioritise epitopes for removal, the epitope clusters were then mapped to the known structure-function features on the molecule. For INF2 evidence from homology modelling (Murgolo N.J. *et al., Proteins: Structure, Function & Genetics* **17** 62-74 (1993)), site directed mutagenesis (Tymms, M.J*. et al*., *Antiviral Res.* **12** 37-48 (1989); McInnes B. *et al*., *J*. *Interferon Res.* **9** 305-314 (1989)), cross-species chimaeric molecules (Ra, N.B.K. *et al*., *J*. *Biol. Chem.* **263** 8943-8952 (1988); Shafferman, A. *et al*. *J*. *Biol*. *Chem*. **262** 6227-6237 (1987)), deletion mutants (Wetzel, R. *et al*., In: *Interferons* New York Academic Press, pp819-823 (1982)) and serological mapping studies (Lydon N.B. *et al*., *Biochemistry* **24** 4131-4141 (1985); Trotta P.P. *et al*.,. In: *The Interferon System* Dianzani F & Rossi G.B (eds.) New York, Raven Press, 1985 pp231-235), suggest epitopes in clusters 1 and 2 are the highest priority targets for removal. With reference to the structural model of Murgolo et al (Murgolo N.J. et al (1993) *ibid.),* cluster 1 featuring 5 potential epitopes encompasses the functionally important helix A and the AB surface loop region of the INF2 molecule. This region is important for the antiviral activity and is involved in binding to the human INF2 receptor structure. Most significantly, epitopes for neutralising antibodies have been mapped to this region, specifically residues 10-11 of the helix A structural element (Lydon *N.B. et al.,* (1985) *ibid.).*

On this basis, cluster 1 epitopes at positions 7-19 and 13-25 were eliminated by substitution of threonine for leucine at position 15 (L₁₅T using single letter codes). This procedure was conducted interactively *in silico* using the MPTver1 package. Similarly, cluster 1 epitope at position 28-41 was eliminated by substitution F₂₇Y. This latter substitution had the concomitant effect of reducing the number of potential binding alleles for the overlapping epitope at position 22-34 from 13 to 11.

Cluster 2 epitopes extend from the AB loop through the helix B region and into the BC loop domain. Neutralising antibodies have been shown to bind in the BC loop (Lydon N.B. *et al.,* (1985) *ibid.;* Trotta P.P. *et al.,.* (1985) *ibid. )* therefore cluster 2 epitope at position 58-70 was also targeted for removal. Substitution N₆₅L reduces potential immunogenicity in this region by eliminating 3 overlapping epitopes encompassing positions 61-77, which collectively are predicted to bind 5 different MHC DR alleles. Additionally; this substitution also reduces the number of different binding alleles at epitope 58-70 from 5 to 3.

Other epitope clusters (e.g. 3-5) map to either buried regions of the molecule or surface regions not involved in receptor binding and hence provide antigenic sites to which antibody responses are largely non neutralising.

Using the above method, an altered INF2 protein sequence was compiled containing 3 substitutions from the starting sequence and is depicted in Figure 2. This sequence is predicted to be significantly less immunogenic with respect to human MHC class II presentation. The areas of reduced immunogenicity focussed to regions of the molecule where antibody mediated neutralisation of the protein has been shown to occur with the potential to limit the clinical efficacy of the molecule as a therapeutic entity.

### Method for construction of altered interferon alpha 2 molecule:

A wild-type INF2 gene was synthesised under contract by Genosys Biotechnologies Ltd (Cambridge, UK). The gene was constructed by PCR using long (80-mer) overlapping synthetic DNA primers and the sequence given in GenBank accession number M29883. The synthetic gene was cloned as a 520bp *Eco*RI-*Hin*dIII restriction fragment into bacterial expression vector pMEX8 (MoBiTec, Gottingen, Germany). The gene sequence was confirmed to be identical to the desired gene using commercially available reagent systems and instructions provided by the supplier (Amersham, Little Chalfont, UK).

Altered (reduced immunogenicity) versions of the protein were constructed by site directed mutagenesis of the wild-type gene in pMEX8. Mutagenesis was conducted using short (18-mer) synthetic oligonucleotides obtained commercially (Genosys, Cambridge, UK) and the "quick-change" procedure and reagents from Stratagene (Cambridge, UK). Following site directed mutagenesis, DNA sequences of selected clones were confirmed as previously.

For expression of recombinant wild-type and recombinant variant INF2 proteins, pMEX8-INF2 expression plasmids were transformed into *E. coli* strain JA221 and cells grown and harvested using standard procedures (Sambrook J., Fritisch E.F. & Maniatis T. (1989) *ibid.*)*.* Recombinant INF2 was prepared essentially as described previously (Grosfeld, H. *et al*., in *Advances in Biotechnological Processes 4,* Mizrahi A. & Van Wezel A. L. eds., pp59-78 Alan R. Liss Inc, New York (1985)) with minor modifications. Briefly, following high speed centrifugation, the supernatant was concentrated by 60% saturation of ammonium sulphate and then chromatographed on a 2.7 x 68cm Sephadex G-75 column equilibrated with PBS plus 0.5M NaCl. Fractions (8ml) were collected at a 1min/ml flow rate. Pooled fractions were further purified using an immunoaffinity column as described previously (Grosfeld, H. *et al*., (1985) *ibid*). Purified proteins were assayed using SDS-PAGE analysis and functional activity (anti-viral activity) determined using biological assays as described previously (Shafferman, *A. et al*., *J. Biol. Chem.* **262** 6227-6237 (1987)).

## Claims

1. A method of rendering human interferon alpha 2 (IFN2) non-immunogenic or less immunogenic to a human compared to unmodified IFN2 when used in vivo the method the method comprising:
(a) determining at least part of the amino acid sequence of mature human IFN2;
(b) identifying potential T-cell epitopes by analysing said sequence for the presence of human MHC class II binding motifs, and
(c) eliminating from said amino acid sequence at least one of said T-cell epitopes identified according to step b), thereby reducing the immunogenicity of IFN2 when exposed to the immune system of a human.

2. A method as claimed in claim 1, wherein the T cell epitopes are identified by computational peptide threading.

3. A method of claim 1 or 2, wherein the identified T-cell epitopes are prioritised using structure-function features of IFN2.

4. A method according to any of the claims I - 3, wherein a T cell epitope is eliminated by substitution of one or more amino acids within the epitope itself.

5. A method of any of the claims 1 - 4, wherein the altered IFN2 molecule has the protein sequence as depicted in Figure 2.

6. An altered human interferon alpha 2 (IFN2) having the protein sequence as depicted in Figure 2 and having a reduced immunogenicity with respect to human MHC class II presentation.

## Patentansprüche

1. Verfahren, um humanes Interferon alpha 2 (IFN2) für einen Menschen, verglichen mit unmodifiziertem IFN2, bei Verwendung *in vivo,* nicht-immunogen oder weniger immunogen zu machen, wobei das Verfahren umfasst:
(a) Bestimmung von mindestens einem Teil der Aminosäuresequenz des reifen humanen IFN2;
(b) Identifizierung potentieller T-Zell-Epitope durch MHC-Klasse-II-Bindungsmotive, und
(c) Eliminierung von mindestens einem der genannten T-Zell-Epitope, die gemäß Schritt b) identifiziert wurden, aus genannter Aminosäuresequenz, um so die Immunisierungskraft von IFN2 zu vermindern, wenn es dem Immunsystem eines Menschen ausgesetzt wird.

2. Verfahren nach Anspruch 1, wobei die T-Zell-Epitope durch computergestütztes Peptid-Threading identifiziert werden.

3. Verfahren nach Anspruch 1 oder 2, wobei die identifizierten T-Zell-Epitope unter Verwendung von Struktur-Funktions-Merkmalen von IFN2 priorisiert werden.

4. Verfahren nach einem der Ansprüche 1-3, wobei ein T-Zell-Epitop durch Substitution von einer oder mehreren Aminosäuren innerhalb des Epitops selbst eliminiert wird.

5. Verfahren nach einem der Ansprüche 1-4, wobei das veränderte IFN2-Molekül die Proteinsequenz wie in Abbildung 2 dargestellt aufweist.

6. Verändertes humanes Interferon alpha 2 (IFN2) mit der in Abbildung 2 dargestellten Proteinsequenz und mit einer verminderten Immunisierungskraft bezogen auf humane MHC-Klasse-II-Präsentation.

## Revendications

1. Procédé consistant à rendre l'interféron alpha 2 (IFN2) humain non immunogène ou moins immunogène vis-à-vis d'un être humain par comparaison avec de l'IFN2 non modifié lors d'une utilisation in vivo, le procédé comprenant :
(a) la détermination d'au moins une partie de la séquence d'acides aminés de l'IFN2 de l'être humain mature ;
(b) l'identification d'épitopes de cellule T potentiels en analysant ladite séquence quant à la présence de motifs de liaison MHC de l'être humain de classe III ; et
(c) l'élimination de ladite séquence d'acides aminés d'au moins l'un desdits épitopes de cellule T identifiés selon l'étape b), d'où ainsi la réduction de l'immunogénicité de l'IFN2 lorsqu'il est exposé au système immunitaire de l'être humain.

2. Procédé selon la revendication 1, dans lequel les épitopes de cellule T sont identifiés au moyen d'un brin de peptide obtenu par calcul.

3. Procédé selon la revendication 1 ou 2, dans lequel les épitopes de cellule T identifiés sont soumis à priorité en utilisant des caractéristiques structure-fonction de l'IFN2.

4. Procédé selon l'une quelconque des revendications 1 à 3, dans lequel un épitope de cellule T est éliminé par substitution d'un ou de plusieurs acides aminés à l'intérieur de l'épitope lui-même.

5. Procédé selon l'une quelconque des revendications 1 à 4, dans lequel la molécule IFN2 altérée comporte la séquence de protéines telle que représentée sur la figure 2.

6. Interféron alpha 2 (IFN2) humain altéré comportant la séquence de protéines telle que représentée sur la figure 2 et présentant une immunogénicité réduite par rapport à une présentation de MHC humain de classe II.


### SEQUENCE LISTING

<110> Biovation Limited
<120> Modifying Protein Immunogenicity
<130> P22184WO
<140> PCT/GB99/04119
   <141> 1999-12-08
<150> GB9826925.1
   <151> 1998-12-08
<150> GB9902139.6
   <151> 1999-02-02
<160> 26
<170> PatentIn Ver. 2.1
<210> 1
   <211> 165
   <212> PRT
   <213> Homo sapiens
<400> 14

Cys Asp Leu Pro Gln Thr His Ser Leu Gly Ser Arg Arg Thr Thr Met
1               5                   10                  15

Leu Leu Ala Gln Met Arg Lys Ile Ser Leu Phe Tyr Cys Leu Lys Asp
            20                  25                  30

Arg His Asp Phe Gly Phe Pro Gln Glu Glu Phe Gly Asn Gln Phe Gln
        35                  40                  45

Lys Ala Glu Thr Ile Pro Val Leu His Glu Met Ile Gln Gln Ile Phe
    50                  55                  60

Leu Leu Phe Ser Thr Lys Asp Ser Ser Ala Ala Trp Asp Glu Thr Leu
65                  70                  75                  80

Leu Asp Lys Phe Tyr Thr Glu Leu Tyr Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Arg Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Leu Ser Thr Asn Leu Gln Glu Ser
145                 150                 155                 160

Leu Arg Ser Lys Glu
                165